# EUROPÄISCHE PATENTSCHRIFT

(11) **EP 1 844 080 B1**
(45) Veröffentlichungstag und Bekanntmachung des Hinweises auf die Patenterteilung: **29.10.2014**
(21) Anmeldenummer: 06707824.6
(22) Anmeldetag: 24.01.2006
(51) Int. Cl.: B01J 8/36, C08F 2/34, C08F 2/01, C08F 6/00, C08J 3/24, A61L 15/60, B01J 8/38, B01J 8/00, F26B 3/08

(54) **VERFAHREN ZUR HERSTELLUNG WASSERABSORBIERENDER POLYMERPARTIKEL DURCH VERTROPFUNGSPOLYMERISATION IN DER GASPHASE**
METHOD FOR PRODUCING WATER-ABSORBENT POLYMER PARTICLES BY DROPLET POLYMERISATION IN A GAS PHASE
PROCEDE DE PRODUCTION DE PARTICULES POLYMERES HYDROABSORBANTES, PAR POLYMERISATION EN GOUTTES EN PHASE GAZEUSE

(30) Priorität: 28.01.2005 DE 102005004296; 24.03.2005 DE 102005014292; 11.01.2006 DE 102006001596
(43) Veröffentlichungstag der Anmeldung: 17.10.2007
(73) Patentinhaber: BASF SE, 67056 Ludwigshafen (DE)
(72) Erfinder: BRUHNS, Stefan, 68165 Mannheim (DE); FRENZ, Volker, 32351 Stemwede (DE); LÖSCH, Dennis, 67122 Altrip (DE); SEIDL, Volker, 68163 Mannheim (DE); STUEVEN, Uwe, 65812 Bad Soden (DE); DÜCKER, Carolin Nadine, 67063 Ludwigshafen (DE); WEISMANTEL, Matthias, 63637 Jossgrund (DE); HEIDE, Wilfried, 67251 Freinsheim (DE); BLEI, Stefan, 68163 Mannheim (DE)
(86) Internationale Anmeldenummer: PCT/EP2006/050419
(87) Internationale Veröffentlichungsnummer: WO 2006/079631

(56) Entgegenhaltungen:
- EP-A- 1 097 946
- EP-A1- 1 424 346
- WO-A-2005/111088
- WO-A1-02/066520
- WO-A1-2006/077054
- US-A- 5 269 980
- US-A- 6 107 432
- F. L. Buchholz, A. T. Graham: "Modern Superabsorbent Polymer Technology", Wiley-VCH, New York ISBN: 0-471-19411-5 page 74,77,78,

## Beschreibung

Die vorliegende Erfindung betrifft ein Verfahren zur Herstellung wasserabsorbierender Polymerpartikel durch Vertropfungspolymerisation in der Gasphase, wobei die Polymerpartikel nach der Polymerisation in einem Wirbelschichttrockner getrocknet werden, die wasserabsorbierenden Polymerpartikel selber, Hygieneartikel, enthaltend diese wasserabsorbierenden Polymerpartikel, sowie eine Vorrichtung zur Durchführung des Verfahrens.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils angegebenen Kombination, sondern auch in anderen Kombinationen verwendbar sind, ohne den Rahmen der Erfindung zu verlassen.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Wasserabsorbierende Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Eigenschaften der wasserabsorbierenden Polymere können über den Vernetzungsgrad eingestellt werden. Mit steigendem Vernetzungsgrad steigt die Gelfestigkeit und sinkt die Absorptionskapazität. Dies bedeutet, dass mit steigender Absorption unter Druck (AUL) die Zentrifugenretentionskapazität (CRC) abnimmt (zu sehr hohen Vernetzungsgraden nimmt auch die Absorption unter Druck wieder ab).

Zur Verbesserung der Anwendungseigenschaften, wie beispielsweise Flüssigkeitsleitfähigkeit (SFC) in der Windel und Absorption unter Druck (AUL), werden wasserabsorbierende Polymerpartikel im allgemeinen nachvernetzt. Dadurch steigt nur der Vernetzungsgrad der Partikeloberfläche, wodurch die Absorption unter Druck (AUL) und die Zentrifugenretentionskapazität (CRC) zumindest teilweise entkoppelt werden können. Diese Nachvernetzung kann in wässriger Gelphase durchgeführt werden. Vorzugsweise werden aber gemahlene und abgesiebte Polymerpartikel (Grundpolymer) an der Oberfläche mit einem Nachvernetzer beschichtet, thermisch nachvernetzt und getrocknet. Dazu geeignete Vernetzer sind Verbindungen, die mindestens zwei Gruppen enthalten, die mit den Carboxylatgruppen des hydrophilen Polymeren kovalente Bindungen bilden können.

Durch Sprühpolymerisation konnten die Verfahrensschritte Polymerisation und Trocknung zusammengefasst werden. Zusätzlich konnte die Partikelgröße durch geeignete Verfahrensführung in gewissen Grenzen eingestellt werden.

Die Herstellung wasserabsorbierender Polymerpartikel durch Sprühpolymerisation wird beispielsweise in EP-A-0 348 180, WO-A-96/40427, DE-A-103 14 466, DE-A-103 40 253 und DE-A-102004024437 beschrieben.

US-5,269,980 beschreibt die Sprühpolymerisation und die Vertropfungspolymerisation.

Aufgabe der vorliegenden Erfindung war die Bereitstellung eines verbesserten Verfahrens zur Herstellung wasserabsorbierender Polymerpartikel.

Gelöst wurde die Aufgabe durch ein Verfahren zur Herstellung wasserabsorbierender Polymerpartikel durch Vertropfungspolymerisation in der Gasphase, dadurch gekennzeichnet, dass die Polymerpartikel nach der Polymerisation getrocknet und wahlweise agglomeriert und/oder nachvernetzt werden, wobei die Trocknung in einer Wirbelschicht durchgeführt wird.

Bei der Vertropfungspolymerisation wird eine Monomerlösung unter Ausbildung von Tropfen in eine Gasphase dosiert. Die Vertropfung der Monomerlösung kann beispielsweise mittels einer Vertropferplatte durchgeführt werden.

Eine Vertropferplatte ist eine Platte mit mindestens einer Bohrung, wobei die Flüssigkeit von oben durch die Bohrung tritt. Die Vertropferplatte bzw. die Flüssigkeit wird in Schwingungen versetzt, wodurch an der Unterseite der Vertropferplatte je Bohrung eine idealerweise monodisperse Tropfenkette erzeugt wird.

Die Anzahl und die Größe der Bohrungen werden gemäß der gewünschten Kapazität und Tropfengröße ausgewählt. Der Tropfendurchmesser beträgt dabei üblicherweise das 1,9fache des Durchmessers der Bohrung. Wichtig ist hierbei, dass die zu vertropfende Flüssigkeit nicht zu schnell durch die Bohrung tritt bzw. der Druckverlust über die Bohrung nicht zu groß ist. Ansonsten wird die Flüssigkeit nicht vertropft, sondern der Flüssigkeitsstrahl wird infolge der hohen kinetischen Energie zerrissen (versprüht). Die Geschwindigkeit, mit der die Monomerlösung durch die Bohrung tritt, beträgt vorzugsweise weniger als 0,2 m/s, besonders bevorzugt weniger als 0,1 m/s, ganz besonders bevorzugt weniger als 0,05 m/s. Der Druckverlust über die Bohrung beträgt vorzugsweise weniger als 1 bar, besonders bevorzugt weniger als 0,5 bar, ganz besonders bevorzugt weniger als 0,3 bar.

Die Vertropferplatte weist üblicherweise mindestens eine, vorzugsweise mindestens 10, besonders bevorzugt mindestens 50, und üblicherweise bis zu 10.000, vorzugsweise bis zu 5.000, besonders bevorzugt bis zu 1.000, Bohrungen auf, wobei die Bohrungen üblicherweise gleichmäßig über die Vertropferplatte verteilt sind, vorzugsweise in der sogenannten Dreiecksteilung, d.h. jeweils drei Bohrungen bilden die Ecken eines gleichseitigen Dreiecks.

Der Durchmesser der Bohrungen wird an die gewünschte Tropfengröße angepasst. Der Durchmesser der Bohrungen beträgt üblicherweise mindestens 50 µm, vorzugsweise mindestens 75 µm, besonders bevorzugt mindestens 100 µm, und üblicherweise bis zu 1.000 µm, vorzugsweise bis zu 600 µm, besonders bevorzugt bis zu 300 µm.

Es kann vorteilhaft sein die Vertropferplatte auf eine Trägerplatte aufzulegen, wobei die Trägerplatte ebenfalls Bohrungen aufweist. Dabei weisen die Bohrungen der Trägerplatte einen größeren Durchmesser auf als die Bohrungen der Vertropferplatte auf und sind so angeordnet, dass sich unter jeder Bohrung der Vertropferplatte eine mit ihr konzentrische Bohrung der Trägerplatte befindet. Diese Anordnung ermöglicht einen schnellen Wechsel der Vertropferplatte, beispielsweise um Tropfen einer anderen Größe zu erzeugen. Ein derartiges System aus Vertropferplatte und Trägerplatte gilt als Vertropferplatte im Sinne dieser Erfindung, d.h. die Unterseite des Systems Vertropferplatte/Trägerplatte ist die Unterseite der Vertropferplatte.

Die Vertropfung kann aber auch mittels pneumatischer Ziehdüsen, Rotation, Zerschneiden eines Strahls oder schnell ansteuerbarer Mikroventildüsen durchgeführt werden.

In eine pneumatische Ziehdüse wird ein Flüssigkeitsstrahl zusammen mit einem Gasstrom durch eine Blende beschleunigt. Über die Gasmenge kann der Durchmesser des Flüssigkeitsstrahls und damit der Tropfendurchmesser beeinflusst werden.

Bei der Vertropfung durch Rotation tritt die Flüssigkeit durch die Öffnungen einer rotierenden Scheibe. Durch die auf die Flüssigkeit wirkende Fliehkraft werden Tropfen definierter Größe abgerissen.

Der austretende Flüssigkeitsstrahl kann aber auch mittels eines rotierenden Messers in definierte Segmente zerschnitten werden. Jedes Segment bildet anschließend einen Tropfen.

Bei Verwendung von Mikroventildüsen werden direkt Tropfen mit definiertem Flüssigkeitsvolumen erzeugt.

Die Polymerpartikel weisen bei der Überführung in den Wirbelschichttrockner, d.h. nach der Polymerisation, einen Restmonomerengehalt von weniger als 20 Gew.-%, vorzugsweise von weniger als 15 Gew.-%, besonders bevorzugt von weniger als 10 Gew.-%, ganz besonders bevorzugt von weniger als 5 Gew.-%, und einen Wassergehalt von 1 bis 35 Gew.-%, vorzugsweise von 5 bis 30 Gew.-%, besonders bevorzugt von 10 bis 25 Gew.-%, ganz besonders bevorzugt 15 bis 20 Gew.-%, auf. Der Wassergehalt wird gemäß der von der EDANA (European Disposables and Nonwovens Association) empfohlenen Testmethode Nr. 430.2-02 "Moisture content" bestimmt. Der Restmonomerengehalt wird gemäß der von der EDANA (European Disposables and Nonwovens Association) empfohlenen Testmethode Nr. 410.2-02 "Residual monomers" bestimmt.

Durch das erfindungsgemäße Verfahren werden wasserabsorbierende Polymerpartikel mit hoher Absorption unter Druck und einem niedrigen Anteil an Extrahierbaren erhalten. Insbesondere weisen die gemäß dem erfindungsgemäßen Verfahren hergestellten wasserabsorbierenden Polymerpartikel bereits nach der Trocknung in der Wirbelschicht eine Absorption unter Druck auf, wie sie üblicherweise erst durch Nachvernetzung erhalten wird.

Möglicherweise werden durch die Polymerisation relativ großer Tropfen in der Gasphase strukturierte Polymerpartikel mit einer ausgeprägten Schale erhalten. Hierbei sorgt die Schale für eine hohe Gelfestigkeit Infolge der niedrigen Vernetzungsdichte im Kern bleibt die Absorption an sich aber hoch. Daher weisen die gemäß dem erfindungsgemäßen Verfahren hergestellten wasserabsorbierenden Polymerpartikel eine hohe Absorption unter Druck auf. Durch mechanische Belastung wird diese Schale zerstört und der Anteil an Extrahierbaren steigt deutlich an. Bei zu schneller Trocknung, beispielsweise durch hohe Temperaturen im Reaktor, wird die Schale dagegen durch den zu schnell austretenden Wasserdampf zerrissen und die Polymerisation durch Lösungsmittelmangel zu schnell beendet. Daher es wichtig, dass die durch Vertropfungspolymerisation erhalten Polymerpartikel schonend nachgetrocknet werden.

Die Polymerpartikel werden im fluidisiertem Zustand aus der Polymerisation in mindestens einen der Verfahrensschritte Trocknung, Agglomeration oder Nachvernetzung überführt.

Besonders bevorzugt werden die wasserabsorbierenden Polymerpartikel beim Transport immer im fluidisierten Zustand gehalten.

Agglomeration und Nachvernetzung können nacheinander oder zeitgleich durchgeführt werden. Die Durchführung von Agglomeration und Nachvernetzung in einem Verfahrensschritt ist bevorzugt.

Die Überführung der Polymerpartikel von der Polymerisation in den mindestens einen Verfahrensschritt Trocknung, Agglomeration oder Nachvernetzung in fluidisiertem Zustand unterliegt keiner Beschränkung.

Im fluidisierten Zustand ist die kinetische Energie der Polymerpartikel größer als das Kohäsions- bzw. Adhäsionspotential zwischen den Polymerpartikeln.

Der fluidisierte Zustand kann durch eine Wirbelschicht erreicht werden. Dabei werden die wasserabsorbierenden Polymerpartikel von unten mittels eines geeigneten Trägergases angeströmt, so dass die Partikel eine Wirbelschicht ausbilden. Die Höhe der Wirbelschicht wird durch Gasmenge und Gasgeschwindigkeit, d.h. über den Druckverlust der Wirbelschicht (kinetische Energie des Gases), eingestellt.

Der fluidisierte Zustand kann aber auch durch eine pneumatische Förderung erzielt werden. Dabei werden die wasserabsorbierenden Polymerpartikel in Transportrichtung mittels eines geeigneten Trägergases angeströmt. Der Transport im fluidisierten Zustand wird ebenfalls durch Gasmenge und Gasgeschwindigkeit, d.h. über die kinetische Energie des Gases, eingestellt.

Die einfachste und bevorzugte Variante ist, dass die wasserabsorbierenden Polymerpartikel durch eine geeignete Verbindung aus dem vorigen Verfahrensschritt direkt in mindestens einen der Verfahrensschritte Trocknung, Agglomeration oder Nachvernetzung fallen. Dabei kann der fluidisierte Zustand über einen weiten Bereich über Durchsatz und Trägergasmenge eingestellt werden. Üblicherweise weisen frei fallende Partikel einen ausreichenden Abstand voneinander und eine ausreichende kinetische Energie auf, so dass ein fluidisierter Zustand vorliegt.

Geeignete Verbindungen sind beispielsweise Rohre, wobei Rohrdurchmesser und Rohrneigung so zu wählen sind, dass die Öffnungen an den Rohrenden in der Senkrechten zumindest teilweise, vorzugsweise zu mindestens 50%, besonders bevorzugt zu mindestens 70%, ganz besonders bevorzugt zu mindestens 90%, überlappen. Besonders bevorzugt wird die Verbindung hergestellt, indem der Polymerisationsreaktor direkt mit dem Wirbelschichtapparat verbunden wird, d.h. formal ein Rohr mit der Länge 0 verwendet wird.

Die Vertropfungspolymerisation wird vorzugsweise in einer laminaren Gasströmung durchgeführt. Eine laminare Gasströmung ist eine Gasströmung, bei der sich die einzelnen Schichten der Strömung nicht vermischen, sondern parallel bewegen. Ein Maß für die Strömungsverhältnisse ist die Reynolds-Zahl (Re). Unterhalb einer kritischen Reynolds-Zahl (Reₖᵣᵢₜ) von 2300 ist die Gasströmung laminar. Die Reynolds-Zahl der laminaren Gasströmung beträgt vorzugsweise weniger als 2000, besonders bevorzugt weniger als 1500, ganz besonders bevorzugt weniger als 1000. Der untere Grenzfall der laminaren Inertgasströmung ist eine ruhende Inertgasatmosphäre (Re = 0), d.h., es wird nicht kontinuierlich Inertgas eingespeist.

Vorzugsweise werden Agglomeration und/oder Nachvernetzung in fluidisiertem Zustand durchgeführt.

Zur Agglomeration und/oder Nachvernetzung wird eine Lösung mit dem Agglomerationshilfsmittel und/oder dem Nachvernetzer auf die wasserabsorbierenden Polymerpartikel aufgesprüht und thermisch getrocknet.

Das Aufsprühen der Lösung kann beispielsweise in Mischern mit bewegten Mischwerkzeugen, wie Schneckenmischer, Paddelmischer, Scheibenmischer, Pflugscharmischer und Schaufelmischer, durchgeführt werden. Geeignete Mischer sind beispielsweise Lödige®-Mischer, Bepex®-Mischer, Nauta®-Mischer, Processall®-Mischer und Schugi®-Mischer. Bevorzugt sind Vertikalmischer. Besonders bevorzugt sind Wirbelschichtapparate.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex®-Trockner und Nara®-Trockner. Überdies können auch Wirbelschichttrockner eingesetzt werden.

Die Trocknung wird vorzugsweise im Mischer selbst durchgeführt, beispielsweise durch Beheizung des Mantels oder Einblasen von Warmluft. In einer besonders bevorzugten Ausführungsform wird die Lösung in einem Wirbelschichtreaktor auf die wasserabsorbierenden Polymerpartikel aufgebracht und im Wirbelschichtreaktor agglomeriert und/oder nachvernetzt.

Die im erfindungsgemäßen Verfahren bevorzugt einsetzbaren Monomerlösungen enthalten
a) mindestens ein ethylenisch ungesättigtes, säuregruppentragendes Monomer,
b) mindestens einen Vernetzer,
c) wahlweise ein oder mehrere mit dem Monomeren a) copolymerisierbare ethylenisch und/oder allylisch ungesättigte Monomere und
d) wahlweise ein oder mehrere wasserlösliche Polymere, auf die die Monomere a), b) und ggf. c) zumindest teilweise aufgepfropft werden können.

Geeignete Monomere a) sind beispielsweise ethylenisch ungesättigte Carbonsäuren, wie Acrylsäure, Methacrylsäure, Maleinsäure, Fumarsäure und Itaconsäure, oder deren Derivate, wie Acrylamid, Methacrylamid, Acrylsäureester und Methacrylsäureester. Besonders bevorzugte Monomere sind Acrylsäure und Methacrylsäure. Ganz besonders bevorzugt ist Acrylsäure.

Die Säuregruppen der Monomere a) sind üblicherweise teilweise neutralisiert, vorzugsweise zu 25 bis 85 mol-%, bevorzugt zu 27 bis 80 mol-%, besonders bevorzugt zu 27 bis 30 mol-% oder 40 bis 75 mol-%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumcarbonat oder Natriumhydrogencarbonat sowie deren Mischungen. Üblicherweise wird die Neutralisation durch Einmischung des Neutralisationsmittels als wässrige Lösung, als Schmelze, oder bevorzugt auch als Feststoff erreicht. Beispielsweise kann Natriumhydroxid mit einem Wasseranteil deutlich unter 50 Gew.-% als wachsartige Masse mit einem Schmelzpunkt oberhalb 23°C vorliegen. In diesem Fall ist eine Dosierung als Stückgut oder Schmelze bei erhöhter Temperatur möglich.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden 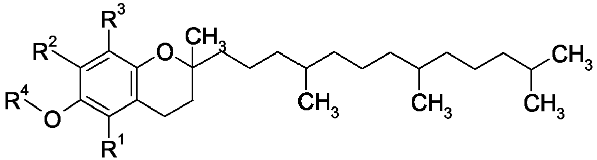 wobei R¹ Wasserstoff oder Methyl, R² Wasserstoff oder Methyl, R³ Wasserstoff oder Methyl und R⁴ Wasserstoff oder ein Säurerest mit 1 bis 20 Kohlenstoffatomen bedeutet.

Bevorzugte Reste für R⁴ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit R¹ = R² = R³ = Methyl, insbesondere racemisches alpha-Tocopherol. R¹ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenden Gehalt an Hydrochinonhalbether verwendet werden.

Die Vernetzer b) sind Verbindungen mit mindestens zwei radikalisch polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP-A-0 530 438 beschrieben, Di- und Triacrylate, wie in EP-A-0 547 847, EP-A-0 559 476, EP-A-0 632 068, WO-A-93/21237, WO-A-03/104299, WO-A-03/104300, WO-A-03/104301 und in DE-A-103 31 450 beschrieben, gemischte Acrylate, die neben Acrylatgruppen weitere ethylenisch ungesättigte Gruppen enthalten, wie in DE-A-103 314 56 und der älteren deutschen Anmeldung mit dem Aktenzeichen 10355401.7 beschrieben, oder Vernetzermischungen, wie beispielsweise in DE-A-195 43 368, DE-A-196 46 484, WO-A-90/15830 und WO-A-02/32962 beschrieben.

Geeignete Vernetzer b) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A-0 343 427 beschrieben sind. Weiterhin geeignete Vernetzer b) sind Pentaerythritoldi-Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufweist.

Besonders vorteilhafte Vernetzer b) sind jedoch Di- und Triacrylate des 3- bis 15-fach ethoxylierten Glyzerins, des 3- bis 15-fach ethoxylierten Trimethylolpropans, des 3-bis 15-fach ethoxylierten Trimethylolethans, insbesondere Di- und Triacrylate des 2-bis 6- fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des 40-fach ethoxylierten Glyzerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine wie sie beispielsweise in WO-A-03/104301 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5- fach ethoxylierten und/oder propoxylierten Glyzerins. Am meisten bevorzugt sind die Triacrylate des 3-bis 5-fach ethoxylierten und/oder propoxylierten Glyzerins. Diese zeichnen sich durch besonders niedrige Restgehalte (typischerweise unter 10 Gew.-ppm) im wasserabsorbierenden Polymer aus und die wässrigen Extrakte der damit hergestellten wasserabsorbierenden Polymere weisen eine fast unveränderte Oberflächenspannung (typischerweise mindestens 0,068 N/m) im Vergleich zu Wasser gleicher Temperatur auf.

Mit den Monomeren a) copolymerisierbare ethylenisch ungesättigte Monomere c) sind beispielsweise Acrylamid, Methacrylamid, Crotonsäureamid, Dimethylaminoethylmethacrylat, Dimethylaminoethylacrylat, Dimethylaminopropylacrylat, Diethylaminopropylacrylat, Dimethylaminobutylacrylat, Dimethylaminoethylmethacrylat, Diethylaminoethylmethacrylat, Dimethylaminoneopentylacrylat und Dimethylaminoneopentylmethacrylat.

Als wasserlösliche Polymere d) können Polyvinylalkohol, Polyvinylpyrrolidon, Stärke, Stärkederivate, Polyglykole oder Polyacrylsäuren, vorzugsweise Polyvinylalkohol und Stärke, eingesetzt werden.

Die Reaktion wird vorzugsweise in Gegenwart eines inerten Trägergases durchgeführt werden, wobei inert bedeutet, dass das Trägergas mit den Bestandteilen der Monomerlösung nicht reagieren kann. Das inerte Trägergas ist vorzugsweise Stickstoff. Der Sauerstoffgehalt des inerten Trägergases beträgt vorteilhaft unter 5 Vol.-%, vorzugsweise unter 2 Vol.-%, besonders bevorzugt unter 1 Vol.-%. Das Trägergas wird vorzugsweise auch für den Wirbelschichtapparat und gegebenenfalls die pneumatische Förderung verwendet.

Das inerte Trägergas kann im Gleichstrom oder im Gegenstrom zu den frei fallenden Tropfen der Monomerlösung durch den Reaktionsraum geführt werden, bevorzugt im Gleichstrom. Vorzugsweise wird das Trägergas nach einem Durchgang zumindest teilweise, bevorzugt zu mindestens 50%, besonders bevorzugt zu mindestens 75%, als Kreisgas in den Reaktionsraum zurückgeführt. Üblicherweise wird eine Teilmenge des Trägergases nach jedem Durchgang ausgeschleust, vorzugsweise bis zu 10%, besonders bevorzugt bis zu 3%, ganz besonders bevorzugt bis zu 1%.

Die Gasgeschwindigkeit wird vorzugsweise so eingestellt, dass die Strömung im Reaktor gerichtet ist, beispielsweise liegen keine der allgemeinen Strömungsrichtung entgegengesetzte Konvektionswirbel vor, und beträgt beispielsweise 0,02 bis 1,5 m/s, bevorzugt 0,05 bis 0,4 m/s.

Die Temperatur im Reaktionsraum beträgt bei der thermisch induzierten Polymerisation vorzugsweise 70 bis 250°C, besonders bevorzugt 80 bis 190°C, ganz besonders bevorzugt 90 bis 140°C.

Die Konzentration der Monomeren a) in der Monomerlösung beträgt üblicherweise 2 bis 80 Gew.-%, vorzugsweise 5 bis 70 Gew.-%, besonders bevorzugt 10 bis 60 Gew.-%.

Die Löslichkeit der Monomeren a) in Wasser beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 50 g/100 g Wasser.

Die bevorzugten Polymerisationsinhibitoren benötigen für eine optimale Wirkung gelösten Sauerstoff. Daher können die Polymerisationsinhibitoren vor der Polymerisation durch Inertisierung, d.h. Durchströmen mit einem inerten Gas, vorzugsweise Stickstoff, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, gesenkt.

Die Polymerisationsinhibitoren können auch durch Absorption, beispielsweise an Aktivkohle, entfernt werden.

Die Monomerlösung wird in Gegenwart von Initiatoren polymerisiert.

Die Initiatoren werden in üblichen Mengen eingesetzt, beispielsweise in Mengen von 0,001 bis 5 Gew.-%, vorzugsweise 0,01 bis 1 Gew.-%, bezogen auf die zu polymerisierenden Monomere.

Als Initiatoren können sämtliche unter den Polymerisationsbedingungen in Radikale zerfallende Verbindungen eingesetzt werden, beispielsweise Peroxide, Hydroperoxide, Wasserstoffperoxid, Persulfate, Azoverbindungen und die sogenannten Redoxinitiatoren. Bevorzugt ist der Einsatz von wasserlöslichen Initiatoren.

Geeignete organische Peroxide sind beispielsweise Acetylacetonperoxid, Methylethylketonperoxid, tert.-Butylhydroperoxid, Cumolhydroperoxid, tert.-Amylperpivalat, tert.-Butylperpivalat, tert.-Butylperneohexanoat, tert.-Butylperisobutyrat, tert.-Butyl-per-2-ethylhexanoat, tert.-Butylperisononanoat, tert.-Butylpermaleat, tert.-Butylperbenzoat, Di-(2-ethylhexyl)peroxydicarbonat, Dicyclohexylperoxydicarbonat, Di-(4-tert.-butylcyclohexyl)peroxydicarbonat, Dimyristilperoxydicarbonat, Diacetylperoxydicarbonat, Allylperester, Cumylperoxyneodecanoat, tert.-Butylper-3,5,5-trimethylhexanoat, Acetylcyclohexylsulfonylperoxid, Dilaurylperoxid, Dibenzoylperoxid und tert.-Amylperneodekanoat.

Bevorzugte Initiatoren sind Azoverbindungen, beispielsweise 2,2'-Azobis-isobutyronitril, 2,2'-Azobis(2,4-dimethylvaleronitril) und 2,2'-Azobis(4-methoxy-2,4-dimethylvaleronitril), insbesondere wasserlösliche Azostarter, beispielsweise 2,2'-Azobis{2-[1-(2-hydroxyethyl)-2-imidazolin-2-yl]propan}dihydrochlorid, 2,2'-Azobis-(2-amidinopropan)dihydrochlorid, 2,2'-Azobis[2-(2-imidazolin-2-yl)propan]dihydrochlorid und 2,2'-Azobis[2-(5-methyl-2-imidazolin-2-yl)propan]dihydrochlorid. Ganz besonders bevorzugt sind 2,2'-Azobis[2-(2-imidazolin-2-yl)propan]dihydrochlorid und 2,2'-Azobis[2-(5-methyl-2-imidazolin-2-yl)propan]dihydrochlorid.

Weiterhin bevorzugte Initiatoren sind außerdem Redoxinitiatoren. Die Redoxinitiatoren enthalten als oxidierende Komponente mindestens eine der oben angegebenen Peroxoverbindungen und als reduzierende Komponente beispielsweise Ascorbinsäure, Glukose, Sorbose, Ammonium- oder Alkalimetallhydrogensulfit, -sulfit, -thiosulfat, - hyposulfit, -pyrosulfit, -sulfid oder Natriumhydroxymethylsulfoxylat. Vorzugsweise verwendet man als reduzierende Komponente des Redoxkatalysators Ascorbinsäure oder Natriumpyrosulfit. Bezogen auf die bei der Polymerisation eingesetzte Menge an Monomeren verwendet man beispielsweise 1 x 10⁻⁵ bis 1 Mol-% der reduzierenden Komponente des Redoxkatalysators.

Besonders bevorzugt wird die Polymerisation durch Einwirkung energiereicher Strahlung ausgelöst, wobei man üblicherweise sogenannte Photoinitiatoren als Initiator verwendet. Hierbei kann es sich beispielsweise um sogenannte α-Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanton-Derivate, Cumarin-Derivate, Benzoinether und deren Derivate, Azoverbindungen, wie die oben genannten Radikalbildner, substituierte Hexaarylbisimidazole oder Acylphosphinoxide, insbesondere 2-Hydroxy-2-methylpropiophenon (Darocure® 1173). Beispiele für Azide sind 2-(N,N-Dimethylamino)-ethyl-4-azidocinnamat, 2-(N,N-Dimethyl-amino)-ethyl-4-azidonaphthylketon, 2-(N,N-Dimethylamino)-ethyl-4-azidobenzoat, 5-Azido-1-naphthyl-2`-(N,N-dimethylamino)ethylsulfon, N-(4-Sulfonylazidophenyl)maleinimid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclohexanon und 2,6-Bis-(p-azidobenzyliden)-4-methylcyclohexanon.

Besonders bevorzugte Initiatoren sind Azoinitiatoren, wie 2,2'-Azobis[2-(2-imidazolin-2-yl)propan]dihydrochlorid und 2,2'-Azobis[2-(5-methyl-2-imidazolin-2-yl)propan]dihydrochlorid, und Photoinitiatoren, wie 2-Hydroxy-2-methylpropiophenon und 1-[4-(2-Hydroxyethoxy)-phenyl]-2-hydroxy-2-methyl-1-propan-1-on, Redoxinitiatoren, wie Natriumpersulfat/ Hydroxymethylsulfinsäure, Ammoniumperoxodisulfat/Hydroxymethylsulfinsäure, Wasserstoffperoxid/Hydroxymethylsulfinsäure, Natriumpersulfat/Ascorbinsäure, Ammoniumperoxodisulfat/Ascorbinsäure und Wasserstoffperoxid/Ascorbinsäure, Photoinitiatoren, wie 1-[4-(2-Hydroxyethoxy)-phenyl]-2-hydroxy-2-methyl-1-propan-1-on, sowie deren Mischungen.

Der pH-Wert der Monomerlösung ist nicht entscheidend. Entsprechend der Produktanforderungen kann aber der pH-Wert des erfindungsgemäßen Polymeren über den pH-Wert der Monomerlösung auf den gewünschten Bereich eingestellt werden. Beispielsweise sollten Polymere für kosmetische Anwendungen einen pH-Wert um 7 aufweisen.

Der Reaktionsraum des Polymerisationsreaktors kann im Überdruck oder im Unterdruck durchgeführt werden, ein Unterdruck von bis zu 100 mbar gegenüber dem Umgebungsdruck ist bevorzugt.

Die Polymerisationsgeschwindigkeit und die Trockengeschwindigkeit weisen üblicherweise unterschiedliche Temperaturabhängigkeiten auf. Dies kann beispielsweise bedeuten, dass die Tropfen trocknen bevor der gewünschte Umsatz erreicht worden ist. Daher ist es vorteilhaft die Reaktionsgeschwindigkeit und die Trockengeschwindigkeit getrennt zu beeinflussen.

Die Trockengeschwindigkeit kann über die relative Feuchte des Inertgases beeinflusst werden. Die relative Feuchte des Inertgases beträgt im allgemeinen weniger als 90%, vorzugsweise weniger als 60%, besonders bevorzugt weniger als 30%. Dabei ist die relative Feuchte der Quotient aus Wasserdampfpartialdruck und maximalem Wasserdampfpartialdruck (Sättigung) bei einer gegebenen Temperatur multipliziert mit 100%.

Die Polymerisationsgeschwindigkeit kann durch Art und Menge des verwendeten Initiatorsystems eingestellt werden.

Vorteilhaft zur Steuerung der Polymerisationsgeschwindigkeit ist die Verwendung von Azoverbindungen oder Redoxinitiatoren als Initiatoren. Das Anspringverhalten der Polymerisation lässt sich mit Azoverbindungen oder Redoxinitiatoren über Auswahl des Initiators, Initiatorkonzentration und Reaktionstemperatur besser steuern als beispielsweise mit reinen Peroxidinitiatoren.

Das Trägergas wird zweckmäßigerweise vor dem Reaktor auf die Reaktionstemperatur vorgewärmt.

Besonders vorteilhaft sind Photoinitiatoren. Bei Verwendung von Photoinitiatoren kann die Trockengeschwindigkeit über die Temperatur auf den gewünschten Wert eingestellt werden, ohne dass damit gleichzeitig die Radikalbildung wesentlich beeinflusst wird. Das Reaktionsabgas, d.h. das den Reaktionsraum verlassende Trägergas, kann beispielsweise in einem Wärmeaustauscher abgekühlt werden. Geeignete Wärmeaustauscher sind direkte Wärmeaustauscher, wie Wäscher, und indirekte Wärmeaustauscher, wie Kondensatoren. Dabei kondensieren Wasser und nicht umgesetztes Monomer. Danach kann das Reaktionsabgas zumindest teilweise wieder aufgewärmt und als Kreisgas in den Reaktor zurückgeführt werden. Vorzugsweise wird das Kreisgas so abgekühlt, dass das abgekühlte Kreisgas den für die Reaktion gewünschten Anteil an Wasserdampf hat. Ein Teil des Reaktionsabgases kann ausgeschleust und durch frisches Trägergas ersetzt werden, wobei im Reaktionsabgas enthaltene nicht umgesetzte Monomere abgetrennt und rückgeführt werden können.

Besonders bevorzugt ist ein Wärmeverbund, das heißt, ein Teil der Abwärme beim Abkühlen des Abgases wird zum Aufwärmen des Kreisgases verwendet.

Die Reaktoren können begleitbeheizt werden. Die Begleitheizung wird dabei so eingestellt, dass die Wandtemperatur mindestens 5°C oberhalb der Reaktorinnentemperatur liegt und die Kondensation an den Reaktorwänden zuverlässig vermieden wird.

Das Reaktionsprodukt wird vorzugsweise in fluidisiertem Zustand in mindestens einen der Verfahrensschritte Trocknung, Agglomeration und/oder Nachvernetzung überführt.

Die Polymerpartikel werden anschließend getrocknet sowie wahlweise agglomeriert und/oder nachvernetzt.

Geeignete Agglomerationshilfsmittel sind Wasser und mit Wasser mischbare organische Lösungsmittel, wie Alkohole, Tetrahydrofuran und Aceton, wobei zusätzlich wasserlösliche Polymere verwendet werden können.

Geeignete Nachvernetzer sind Verbindungen, die mindestens zwei Gruppen enthalten, die mit den Carboxylatgruppen des Hydrogels kovalente Bindungen bilden können. Geeignete Verbindungen sind beispielsweise Alkoxysiliylverbindungen, Polyaziridine, Polyamine, Polyamidoamine, Di- oder Polyglycidylverbindungen, wie in EP-A-0 083 022, EP-A-0 543 303 und EP-A-0 937 736 beschrieben, di- oder polyfunktionelle Alkohole, wie in DE-C-33 14 019, DE-C-35 23 617 und EP-A-0 450 922 beschrieben, oder ß-Hydroxyalkylamide, wie in DE-A-102 04 938 und US-6,239,230 beschrieben.

Des weiteren sind in DE-A-40 20 780 zyklische Karbonate, in DE-A-198 07 502 2-Oxazolidon und dessen Derivate, wie 2-Hydroxyethyl-2-oxazolidon, in DE-A-198 07 992 Bis- und Poly-2-oxazolidinone, in DE-A-198 54 573 2-Oxotetrahydro-1,3-oxazin und dessen Derivate, in DE-A-198 54 574 N-Acyl-2-Oxazolidone, in DE-A-102 04 937 zyklische Harnstoffe, in DE-A-103 34 584 bizyklische Amidacetale, in EP-A-1 199 327 Oxetane und zyklische Harnstoffe und in WO-A-03/031482 Morpholin-2,3-dion und dessen Derivate als geeignete Nachvernetzer beschrieben.

In einer bevorzugten Ausführungsform der vorliegenden Erfindung wird im laminaren Gleichstrom polymerisiert und im turbulenten Gasstrom getrocknet. Vorzugsweise wird die Polymerisation durch Photoinitiatoren gestartet. Die Tropfen werden vorzugsweise in laminarer Inertgasströmung an den Strahlenquellen zur Initiierung der Polymerisation vorbeigeführt. Die Strahlenquellen werden selbstverständlich außerhalb der Reaktionszone angeordnet. Die laminare Strömung verhindert den Kontakt der Monomertropfen untereinander und damit ein unkontrolliertes Verkleben. Die Inertgastemperatur beträgt vorzugsweise weniger als 80°C, besonders bevorzugt weniger als 60°C, ganz besonders bevorzugt weniger als 40°C, und üblicherweise nicht weniger als 20°C.

Nach einem Monomerumsatz von üblicherweise mindestens 50%, vorzugsweise mindestens 60%, besonders bevorzugt mindestens 70%, ganz besonders bevorzugt mindestens 80%, sind die wasserabsorbierenden Polymerpartikel hinreichend mechanisch stabil und werden im turbulenten Gasstrom getrocknet. Dazu wird dem Reaktor ein vorgewärmter Inertgasstrom zugeführt, wobei die Inertgasmenge so gewählt wird, dass eine turbulente Inertgasströmung entsteht. Die turbulente Gasströmung weist eine Reynolds-Zahl (Re) von über 2300, vorzugsweise von über 3000, besonders bevorzugt von über 4000, ganz besonders bevorzugt von über 5000, auf. Der turbulente Inertgasstrom verbessert den Wärmeübergang und beschleunigt die Trocknung. Das zur Trocknung verwendete Inertgas wird zweckmäßigerweise vor dem Reaktor auf eine Temperatur von 70 bis 250°C, vorzugsweise 90 bis 190°C, besonders bevorzugt 110 bis 160°C, vorgewärmt.

Der Übergang von Polymerisation zu Trocknung erfolgt hierbei durch den freien Fall der wasserabsorbierenden Polymerpartikel von dem oberen in den unteren Teil des Reaktors. Im unteren Teil des Reaktors werden die wasserabsorbierende Polymerpartikel getrocknet und der Monomerumsatz erhöht.

Durch die Kombination Photopolymerisation in laminarer Inertgasströmung und Trocknung in turbulenter Inertgasströmung ist es möglich Polymerisation und Trocknung in einem Apparat durchzuführen und gleichzeitig Polymerisation und Trocknung zu entkoppeln und getrennt zu optimieren.

Die durch Vertropfungspolymerisation in der Gasphase, insbesondere durch thermisch induzierte Polymerisation, herstellbaren Polymerpartikel haben eine im wesentlichen kontinuierliche Oberfläche, die Vertiefungen aufweisen kann. Die im wesentlichen kontinuierliche Oberfläche ist durch die Tropfenform der vertropften Monomerlösung bedingt. Die Monomeren im Tropfen beginnen von der Tropfenoberfläche her zu polymerisieren, wobei sich eine noch weiche, verformbare Polymerhaut ausbildet. Gleichzeitig beginnt Wasser aus dem Tropfen heraus zu verdampfen, wodurch die Polymerhaut aufgebläht wird. Hierbei kann die Polymerhaut gelegentlich auch aufreißen, was zu Störungen der kontinuierlichen Oberfläche führen kann. Durch dieses Aufblähen wird die Porösität und damit die Anquellgeschwindigkeit der Polymerpartikel erhöht. Im weiteren Verlauf der Polymerisation können die Polymerpartikel aufgrund des Masseverlustes durch Verdampfung, sofern das Polymer noch plastisch ist, kollabieren, wodurch Polymerpartikel mit einer im wesentlichen kontinuierlichen aber auch gefalteten Oberfläche entstehen.

Infolge der relativ großen Tropfengröße bei der Vertropfung trocknen die Tropfen während der Polymerisation nur langsam. Dadurch werden Polymerpartikel mit hohem Wassergehalt und hohem Monomerumsatz erhalten. Die Polymerpartikel weisen eine hohe Absorption unter Druck und nur geringe extrahierbare Anteile auf. Die vorteilhaften Eigenschaften werden aber bei mechanischer Belastung, beispielsweise durch Nachtrocknung im Schaufeltrockner oder Pflugscharmischer, wieder zerstört. Daher ist es wichtig, dass die Nachtrocknung möglichst schonend, beispielsweise in einem Wirbelschichttrockner durchgeführt wird.

Die wasserabsorbierenden Polymerpartikel weisen typischerweise eine Zentrifugenretentionskapazität (CRC) von 20 bis 80 g/g, vorzugsweise mindestens 30 g/g, besonders bevorzugt mindestens 40 g/g, auf. Die Zentrifugenretentionskapazität (CRC) wird gemäß der von der EDANA (European Disposables and Nonwovens Association) empfohlenen Testmethode Nr. 441.2-02 "Centrifuge retention capacity" bestimmt.

Die wasserabsorbierenden Polymerpartikel weisen typischerweise eine Absorption unter Druck 0,3 psi (2,07 kPa) von 10 bis 30 g/g, vorzugsweise mindestens 15 g/g, besonders bevorzugt mindestens 20 g/g, auf. Die Absorption unter Druck (AUL) wird gemäß der von der EDANA (European Disposables and Nonwovens Association) empfohlenen Testmethode Nr. 442.2-02 "Absorption under pressure" bestimmt.

Die wasserabsorbierenden Polymerpartikel weisen typischerweise weniger als 15 Gew.-%, vorzugsweise weniger als 10 Gew.-%, besonders bevorzugt weniger als 5 Gew.-%, Extrahierbare auf. Die Extrahierbaren werden gemäß der von der EDANA (European Disposables and Nonwovens Association) empfohlenen Testmethode Nr. 470.2-02 "Extractables" bestimmt.

Die wasserabsorbierenden Polymerpartikel weisen typischerweise einen Wassergehalt von weniger als 15 Gew.-%, vorzugsweise weniger als 10 Gew.-%, besonders bevorzugt weniger als 5 Gew.-%, auf. Der Wassergehalt wird gemäß der von der EDANA (European Disposables and Nonwovens Association) empfohlenen Testmethode Nr. 430.2-02 "Moisture content" bestimmt.

Ein weiterer Gegenstand der vorliegenden Erfindung ist eine Vorrichtung zur Herstellung von Polymeren durch Vertropfungspolymerisation in der Gasphase, umfassend
i) einen beheizbaren Reaktionsraum,
ii) mindestens eine Vorrichtung zur Tropfenerzeugung im oberen Bereich des Reaktionsraumes i),
iii) wahlweise mindestens eine Trägergaszuführung, vorzugsweise im oberen Teil des Reaktionsraumes i),
iv) wahlweise mindestens eine Trägergasvorheizung,
v) wahlweise mindestens eine Trägergasabführung, vorzugsweise im unteren Teil des Reaktionsraumes i),
vi) wahlweise mindestens eine Vorrichtung zur Rückführung zumindest eines Teil des abgeführten Trägergases aus der Trägergasabführung v) zur Trägergaszuführung iii),
vii) wahlweise mindestens eine Strahlenquelle, vorzugsweise im oberen Teil des Reaktionsraumes i),
viii) ein Wirbelschichtapparat außerhalb des Reaktionsraumes i),
ix) wahlweise eine Vorrichtung zum Transport wasserabsorbierender Polymerpartikel im fluidisierten Zustand vom Reaktionsraum i) in den Wirbelschichtapparat viii),
x) wahlweise mindestens eine Vorrichtung zur Agglomeration oder Nachvernetzung und
xi) wahlweise mindestens eine Vorrichtung zum Transport wasserabsorbierender Polymerpartikel im fluidisierten Zustand vom vorigen Verfahrensschritt in die Agglomeration und/oder Nachvernetzung x),
wobei der obere Bereich des Reaktionsraumes die oberen 50%, vorzugsweise die oberen 30%, besonders die oberen 10%, des Reaktionsraumvolumens und der untere Bereich des Reaktionsraumes die unteren 50%, vorzugsweise die unteren 30%, besonders die unteren 10%, des Reaktionsraumvolumens sind und die Vorrichtung eine Verbindung zwischen Polymerisation und mindestens einem der Verfahrensschritte Trocknung, Agglomeration oder Nachvernetzung aufweist, in der die Polymerpartikel im fluidisierten Zustand transportiert werden können.

Die Vorrichtung vi) umfasst beispielsweise einen Verdichter, insbesondere einen Ventilator, eine Mengenmessung und ein regelbares Ventil. Der Verdichter erhöht den Druck des Trägergases und ermöglicht so die Rückführung zur Trägergaszuführung iii). Über Mengenmessung und Ventil kann die rückgeführte Trägergasmenge eingestellt werden.

Die Vorrichtungen ix) und xi) können beispielsweise ein Wirbelschichtapparat oder eine pneumatische Förderung sein. Ebenso ist es möglich, dass aufeinanderfolgende Verfahrensschritte übereinander angeordnet werden, so dass die wasserabsorbierenden Polymerpartikel aus dem vorigen in den nachfolgenden Verfahrenschritt fallen können. In diesem Fall ist die Vorrichtung ix) oder xi) die Verbindung zwischen den Verfahrensschritten.

Die Vorrichtung zur Agglomeration und/oder Nachvernetzung ist vorzugsweise ein Wirbelschichtapparat.

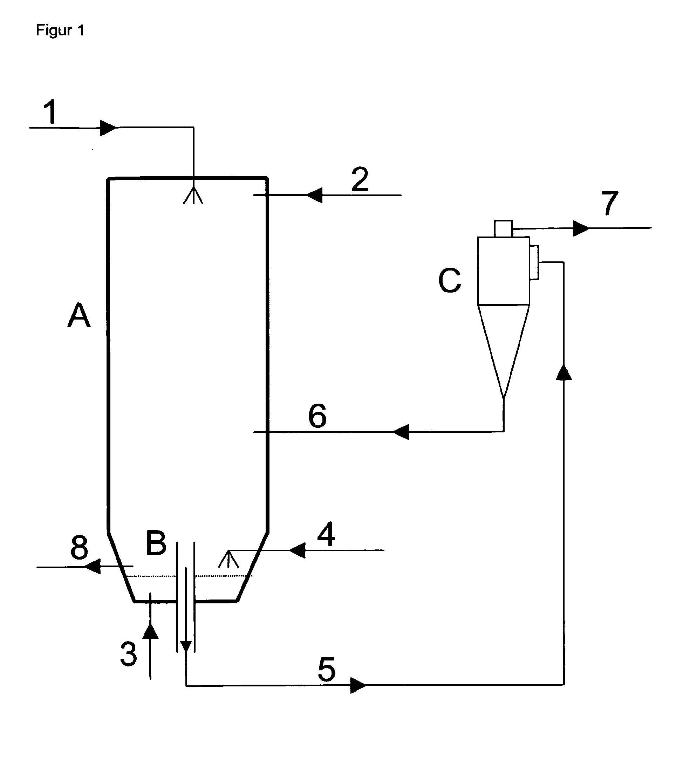

Eine bevorzugte Ausführungsform ist in Figur 1 abgebildet. Dabei haben die Bezugszeichen die folgende Bedeutung:
A: Vertropfungsreaktor
B: integrierter Wirbelschichtapparat
C: Zyklon
1: Zulauf
2: Trägergas (für Reaktion)
3: Trägergas (für Wirbelschicht)
4: Agglomerationshilfsmittel/Nachvernetzer
5: Abgas und Feinstaub
6: Feinstaubrückführung
7: Abgas
8: Produktabzug

In Figur 1 sind Reaktionsraum i) und Wirbelschichttrocknung viii) übereinander angeordnet. Im Wirbelschichtapparat kann zusätzlich agglomeriert und/oder nachvernetzt werden. Mit dem Trägergas mitgerissene Feinstaubanteile werden in einem Zyklon abgeschieden und rückgeführt.

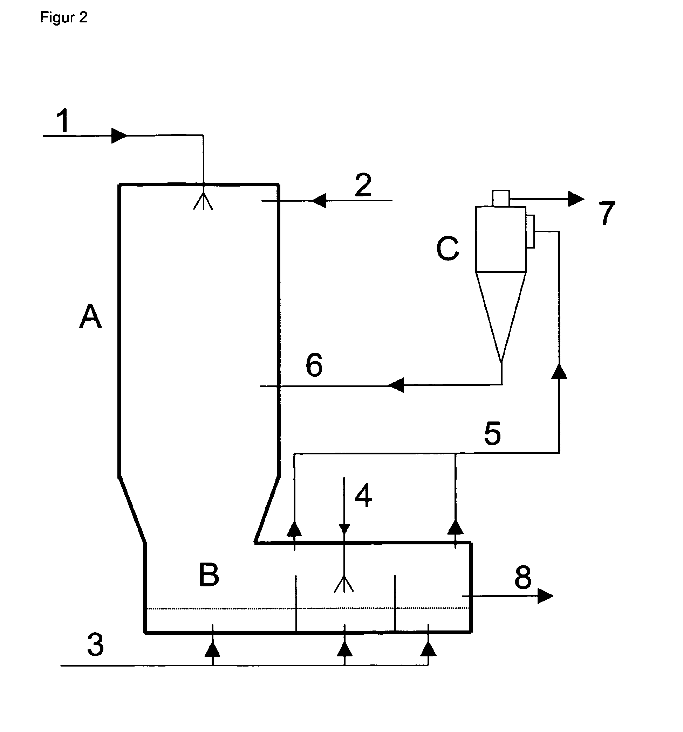

Eine weitere bevorzugte Ausführungsform ist in Figur 2 abgebildet. Dabei haben die Bezugszeichen die obengenannten Bedeutungen, wobei B eine Wirbelschichtkaskade ist.

In Figur 2 ist die Transportvorrichtung xi) ein Wirbelschichtapparat. Über Überlaufwehre gelangen die wasserabsorbierenden Polymerpartikel in einen zweiten und dritten Wirbelschichtapparat. Der zweite und dritte Wirbelschichtapparat ist die Vorrichtung zur Agglomeration und/oder Nachvernetzung x). Über die Höhe der Wehre können die Verweilzeiten eingestellt werden. Vorzugsweise werden in den hintereinander geschalteten Wirbelschichtapparaten unterschiedliche Temperaturen eingestellt, wobei die Temperaturen dabei individuell den in den Wirbelschichten durchzuführenden Aufgaben angepasst werden.

In Figur 2 sind das Mischen der wasserabsorbierenden Polymerpartikel mit dem Agglomerationshilfsmittel und/oder Nachvernetzungsmittel und die thermische Nachbehandlung getrennt. Selbstverständlich können auch beide Schritte in einem Wirbelschichtapparat durchgeführt werden.

Wie Figur 1 und 2 beispielhaft zeigen, ermöglicht das erfindungsgemäße Verfahren auf einfache Weise die Rückführung von Feinanteilen.

Die in den Figuren 1 und 2 dargestellten Ausführungsformen sind nur Beispiele für die erfindungsgemäßen Vorrichtungen zur Vertropfungspolymerisation und schränken die erfindungsgemäße technische Lehre in keiner Weise ein.

Zur Bestimmung der Güte der Nachvernetzung werden die getrockneten wasserabsorbierenden Polymerpartikel mit den nachfolgend beschriebenen Testmethoden geprüft.

### Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymere werden vor der Messung gut durchmischt.

### Wassergehalt

Der Wassergehalt der wasserabsorbierenden Polymerpartikel wird gemäß der von der EDANA (European Disposables and Nonwovens Association) empfohlenen Testmethode Nr. 430.2-02 "Moisture content" bestimmt.

### Zentrifugenretentionskapazität (CRC Centrifuge Retention Capacity)

Die Zentrifugenretentionskapazität der wasserabsorbierenden Polymerpartikel wird gemäß der von der EDANA (European Disposables and Nonwovens Association) empfohlenen Testmethode Nr. 441.2-02 "Centrifuge retention capacity" bestimmt.

### Absorption unter Druck (AUL Absorbency Under Load)

Die Absorption unter Druck der wasserabsorbierenden Polymerpartikel wird gemäß der von der EDANA (European Disposables and Nonwovens Association) empfohlenen Testmethode Nr. 442.2-02 Absorption under pressure" bestimmt.

### Extrahierbare (Extractables)

Die extrahierbaren Anteile der wasserabsorbierenden Polymerpartikel werden gemäß der von der EDANA (European Disposables and Nonwovens Association) empfohlenen Testmethode Nr. 470.2-02" Extractables" bestimmt.

Die EDANA-Testmethoden sind beispielsweise erhältlich bei der European Disposables and Nonwovens Association, Avenue Eugène Plasky 157, B-1030 Brüssel, Belgien.

### Beispiele:

### Beispiel 1

12 kg Natriumacrylat (37,5 gew.-%ige Lösung in Wasser) und 1,1 kg Acrylsäure wurden mit 3 kg Wasser und 9 g 15-fach ethoxiliertem Trimethylolpropantriacrylat gemischt. Die Lösung wurde nach Zugabe von Initiatoren in einen erwärmten, mit Stickstoffatmosphäre gefüllten Vertropfungsturm vertropft (170°C, 12m Höhe, 2m Breite, Gasgeschwindigkeit 0,1 m/s im Gleichstrom). Die Dosiergeschwindigkeit betrug 16 kg/h. Die Vertropferplatte wies 37 Bohrungen ä 170 µm auf. Der Durchmesser der Vertropferplatte betrug 65 mm. Als Initiatoren wurden 2,2-Azobis[2-(2-imidazolin-2-yl)propan]dihydrochlorid und 2,2-Azobis(2-amidinopropan)dihydrochlorid verwendet. Die Konzentration der Initiatoren betrug jeweils 0,2 Gew.-%, bezogen auf das Monomer. Die Initiatoren wurden kurz vor dem Vertropfer in einer 2%igen wässrigen Lösung über einen statischen Mischer mit der Monomerlösung gemischt.

Die am Boden des Vertropfungsturms anfallenden wasserabsorbierenden Polymerpartikel hatten einen Wassergehalt von 16,5 Gew.-%.

Die Polymerpartikel wurde zwei Stunden bei 80°C in einem Wirbelschichttrockner getrocknet.

Die getrockneten, wasserabsorbierenden Polymerpartikel hatten folgende Eigenschaften:

| | |
|---|---|
| CRC: | 56,5 g/g |
| AUL0.3psi | 20,5 g/g |
| Extrahierbare | 10,6 Gew.-% |
| Wassergehalt | 3,5 Gew.-% |

### Beispiel 2 (Vergleich)

Es wurde verfahren wie unter Beispiel 1. Die am Boden des Vertropfungsturms anfallenden wasserabsorbierenden Polymerpartikel wurde in einem Pflugscharmischer getrocknet.

Die getrockneten, wasserabsorbierenden Polymerpartikel hatten folgende Eigenschaften:

| | |
|---|---|
| CRC: | 52,7 g/g |
| AUL0.3psi | 14,4 g/g |
| Extrahierbare | 14,6 Gew.-% |
| Wassergehalt | 3,7 Gew.-% |

## Patentansprüche

1. Verfahren zur Herstellung wasserabsorbierender Polymerpartikel durch Vertropfungspolymerisation in der Gasphase, **dadurch gekennzeichnet, dass** die Polymerpartikel nach der Polymerisation getrocknet und wahlweise agglomeriert und/oder nachvernetzt werden, wobei die Trocknung in einer Wirbelschicht durchgeführt wird und die Polymerpartikel in fluidisiertem Zustand aus der Polymerisation in mindestens einen der Verfahrensschritte Trocknung, Agglomeration und Nachvernetzung überführt werden.

2. Verfahren gemäß Anspruch 1, **dadurch gekennzeichnet, dass** die Polymerpartikel bei der Überführung in den Trockner einen Restmonomerengehalt von weniger als 15 Gew.-% und einen Wassergehalt von 5 bis 30 Gew.-% aufweisen.

3. Verfahren gemäß Anspruch 2, **dadurch gekennzeichnet, dass** der fluidisierte Zustand durch eine Wirbelschicht bewirkt wird.

4. Verfahren gemäß Anspruch 2, **dadurch gekennzeichnet, dass** der fluidisierte Zustand durch eine pneumatische Förderung bewirkt wird.

5. Verfahren gemäß Anspruch 2, **dadurch gekennzeichnet, dass** der fluidisierte Zustand durch freien Fall bewirkt wird.

6. Verfahren gemäß einem der Ansprüche 1 bis 5, **dadurch gekennzeichnet, dass** sich die Polymerpartikel während Agglomeration und/oder Nachvernetzung in fluidisiertem Zustand befinden.

7. Verfahren gemäß Anspruch 6, **dadurch gekennzeichnet, dass** die Agglomeration und/ oder Nachvernetzung in einer Wirbelschicht durchgeführt wird.

8. Verfahren gemäß einem der Ansprüche 1 bis 7, **dadurch gekennzeichnet, dass** die Polymerisation durch Photoinitiatoren ausgelöst wird.

9. Verfahren gemäß Anspruch 8, **dadurch gekennzeichnet, dass** die Polymerisation bis zu einem Monomerumsatz von mindestens 50% bei einer Gastemperatur von weniger als 80°C durchgeführt wird.

10. Verfahren gemäß einem der Ansprüche 1 bis 9, **dadurch gekennzeichnet, dass** die Vertropfung mittels einer schwingenden Platte mit mindestens einer Bohrung durchgeführt wird, Monomerlösung von oben nach unten durch die Bohrung tritt und der Druckverlust über die Bohrung weniger als 1 bar beträgt.

11. Verfahren gemäß einem der Ansprüche 1 bis 10, **dadurch gekennzeichnet, dass** die Vertropfung mittels einer schwingenden Platte mit mindestens einer Bohrung durchgeführt wird und eine Monomerlösung mit einer Geschwindigkeit von weniger als 0,2 m/s von oben nach unten durch die Bohrung tritt.

12. Vorrichtung zur Herstellung wasserabsorbierender Polymerpartikel durch Vertropfungspolymerisation, wobei die Polymerpartikel nach der Polymerisation getrocknet und wahlweise agglomeriert und/oder nachvernetzt werden, **dadurch gekennzeichnet, dass** die Vorrichtung zur Trocknung ein Wirbelschichtapparat ist und dass die Vorrichtung eine Verbindung zwischen Polymerisation und mindestens einem der Verfahrensschritte Trocknung, Agglomeration oder Nachvernetzung aufweist, in der die Polymerpartikel im fluidisierten Zustand transportiert werden können.

13. Vorrichtung gemäß Anspruch 12, wobei die Vorrichtung eine Platte mit mindestens einer Bohrung zur Tropfenerzeugung aufweist.

14. Vorrichtung gemäß Anspruch 12, wobei die Verbindung ein Wirbelschichtapparat ist.

15. Vorrichtung gemäß Anspruch 12, wobei die Verbindung eine pneumatische Förderung ist.

16. Vorrichtung gemäß Anspruch 12, wobei die Verbindung ein Rohr ist, wobei Rohrdurchmesser und Rohrneigung so zu wählen sind, dass sich die Öffnungen an den Rohrenden in der Senkrechten zumindest teilweise überlappen.

17. Vorrichtung gemäß einem der Ansprüche 12 bis 16, wobei die Agglomeration und/oder Nachvernetzung in einem Wirbelschichtapparat durchgeführt wird.

## Claims

1. A process for producing water-absorbing polymeric particles by dropletization polymerization in the gas phase, which comprises the polymeric particles after the polymerization being dried and selectively agglomerated and/or postcrosslinked, the drying being carried out in a fluidized bed and the polymeric particles being transferred in the fluidized state from the polymerization into at least one of the process steps of drying, agglomeration and postcrosslinking.

2. The process according to claim 1 wherein the polymeric particles transferring into the dryer have a residual monomer content of less than 15% by weight and a water content in the range from 5% to 30% by weight.

3. The process according to claim 2 wherein the fluidized state is brought about by a fluidized bed.

4. The process according to claim 2 wherein the fluidized state is brought about by pneumatic conveying.

5. The process according to claim 2 wherein the fluidized state is brought about by free fall.

6. The process according to any one of claims 1 to 5 wherein the polymeric particles are in a fluidized state during agglomeration and/or postcrosslinking.

7. The process according to claim 6 wherein the agglomeration and/or postcrosslinking is carried out in a fluidized bed.

8. The process according to any one of claims 1 to 7 wherein the polymerization is initiated by photoinitiators.

9. The process according to claim 8 wherein the polymerization is carried out up to a monomer conversion of at least 50% at a gas temperature of less than 80°C.

10. The process according to any one of claims 1 to 9 wherein dropletization is effected by means of an oscillating plate having at least one drilled hole, monomer solution passes downwardly through the drilled hole and the pressure drop across the drilled hole is less than 1 bar.

11. The process according to any one of claims 1 to 10 wherein dropletization is effected by means of an oscillating plate having at least one drilled hole, and a monomer solution passes downwardly through the drilled hole at a speed of less than 0.2 m/s.

12. Apparatus for producing water-absorbing polymeric particles by a dropletization polymerization in which the polymeric particles after the polymerization are dried and selectively agglomerated and/or postcrosslinked, wherein the apparatus for drying is a fluidized bed apparatus and wherein the apparatus comprises a connection which connects the polymerization and at least one of the process steps of drying, agglomeration or postcrosslinking and in which the polymeric particles can be transported in a fluidized state.

13. The apparatus according to claim 12 comprising a plate having at least one drilled hole for generating droplets.

14. The apparatus according to claim 12 wherein the connection is a fluidized bed apparatus.

15. The apparatus according to claim 12 wherein the connection is a pneumatic conveyor.

16. The apparatus according to claim 12 wherein the connection is a tube whose diameter and inclination are such that the openings at the ends of the tube partially or completely overlap in the vertical.

17. The apparatus according to any one of claims 12 to 16 wherein the agglomeration and/or postcrosslinking is carried out in a fluidized bed apparatus.

## Revendications

1. Procédé de fabrication de particules polymères absorbant l'eau par polymérisation en gouttes en phase gazeuse, **caractérisé en ce que** les particules polymères sont séchées après la polymérisation et éventuellement agglomérées et/ou post-réticulées, le séchage étant réalisé dans un lit fluidisé et les particules polymères étant transférées à un état fluidisé de la polymérisation dans au moins une des étapes de procédé séchage, agglomération et post-réticulation.

2. Procédé selon la revendication 1, **caractérisé en ce que** les particules polymères présentent lors du transfert dans le séchoir une teneur résiduelle en monomères inférieure à 15 % en poids et une teneur en eau de 5 à 30 % en poids.

3. Procédé selon la revendication 2, **caractérisé en ce que** l'état fluidisé est créé par un lit fluidisé.

4. Procédé selon la revendication 2, **caractérisé en ce que** l'état fluidisé est créé par un transport pneumatique.

5. Procédé selon la revendication 2, **caractérisé en ce que** l'état fluidisé est créé par chute libre.

6. Procédé selon l'une quelconque des revendications 1 à 5, **caractérisé en ce que** les particules polymères se trouvent à un état fluidisé pendant l'agglomération et/ou la post-réticulation.

7. Procédé selon la revendication 6, **caractérisé en ce que** l'agglomération et/ou la post-réticulation sont réalisées dans un lit fluidisé.

8. Procédé selon l'une quelconque des revendications 1 à 7, **caractérisé en ce que** la polymérisation est déclenchée par des photoinitiateurs.

9. Procédé selon la revendication 8, **caractérisé en ce que** la polymérisation est réalisée jusqu'à une conversion des monomères d'au moins 50 % à une température du gaz inférieure à 80 °C.

10. Procédé selon l'une quelconque des revendications 1 à 9, **caractérisé en ce que** la formation des gouttes est réalisée au moyen d'une plaque oscillante comprenant au moins un alésage, la solution de monomères traversant l'alésage du haut vers le bas et la perte de charge dans l'alésage étant inférieure à 1 bar.

11. Procédé selon l'une quelconque des revendications 1 à 10, **caractérisé en ce que** la formation des gouttes est réalisée au moyen d'une plaque oscillante comprenant au moins un alésage et une solution de monomères traverse l'alésage du haut vers le bas à une vitesse inférieure à 0,2 m/s.

12. Dispositif pour la fabrication de particules polymères absorbant l'eau par polymérisation en gouttes, les particules polymères étant séchées après la polymérisation et éventuellement agglomérées et/ou post-réticulées, **caractérisé en ce que** le dispositif pour le séchage est un appareil à lit fluidisé et **en ce que** le dispositif comprend une connexion entre la polymérisation et au moins une des étapes de procédé séchage, agglomération ou post-réticulation, dans laquelle les particules polymères peuvent être transportées à l'état fluidisé.

13. Dispositif selon la revendication 12, dans lequel le dispositif comprend une plaque comprenant au moins un alésage pour la formation de gouttes.

14. Dispositif selon la revendication 12, dans lequel la connexion est un appareil à lit fluidisé.

15. Dispositif selon la revendication 12, dans lequel la connexion est un transport pneumatique.

16. Dispositif selon la revendication 12, dans lequel la connexion est un tube, le diamètre du tube et l'inclinaison du tube étant choisis de sorte que les ouvertures aux extrémités du tube se chevauchent au moins en partie à la verticale.

17. Dispositif selon l'une quelconque des revendications 12 à 16, dans lequel l'agglomération et/ou la post-réticulation sont réalisées dans un appareil à lit fluidisé.
